# EUROPÄISCHE PATENTANMELDUNG

(11) **EP 4 458 300 A1**
(43) Veröffentlichungstag der Anmeldung: **06.11.2024**
(21) Anmeldenummer: 23171927.9
(22) Anmeldetag: 05.05.2023
(51) Int. Cl.: A61B 18/12

(54) **ELEKTROCHIRURGISCHES SYSTEM UND VERFAHREN ZU DESSEN BETRIEB**

(71) Anmelder: Erbe Elektromedizin GmbH, 72072 Tübingen (DE)
(72) Erfinder: Dierl, Christof, 71149 Bondorf (DE); Sauter, Michael, 72461 Albstadt (DE); Frasch, Thomas, 72127 Kusterdingen (DE); Kegreiss, Marc, 72108 Rorrenburg (DE)
(74) Vertreter: Rüger Abel Patentanwälte PartGmbB

(57) **Zusammenfassung**

Die Erfindung betrifft ein elektrochirurgisches System (10) sowie ein Verfahren zu dessen Betrieb. Das elektrochirurgische System (10) weist ein Versorgungsgerät (11) sowie ein daran angeschlossenes elektrochirurgisches Instrument (12) mit wenigstens einer Instrumentenelektrode (14) auf. Mittels der Instrumentenelektrode (14) kann biologisches Gewebe (15) eines Patienten behandelt werden, beispielsweise durch Ausbildung eines zwischen der Instrumentenelektrode (14) und dem Gewebe (15) gebildeten Funkens (F). Das Versorgungsgerät (11) wertet wenigstens einen Betriebsparameter (OP) eines Betriebsstromkreises (17) aus, zudem das elektrochirurgische Instrument (12) und das behandelte Gewebe (15) gehören und der durch das Versorgungsgerät (11) mit einer elektrischen Leistung versorgt wird. Ein Gewebetyp (TT) des behandelten Gewebes (15) wird durch Auswertung wenigstens eines Betriebsparameters (OP) erkannt. Daraufhin kann wenigstens ein elektrischer Parameter der bereitgestellten elektrischen Leistung angepasst bzw. verändert werden, so dass die dem elektrochirurgischen Instrument (12) bereitgestellte elektrische Leistung stets optimiert ist für den Gewebetyp (TT) des aktuell behandelten Gewebes (15) .

## Beschreibung

Die Erfindung betrifft ein elektrochirurgisches System und ein Verfahren zu dessen Betrieb. Das elektrochirurgische System weist ein Versorgungsgerät sowie ein elektrochirurgisches Instrument mit wenigstens einer Instrumentenelektrode auf. Das elektrochirurgische Instrument ist zur Behandlung von biologischem Gewebe eingerichtet. Während der Behandlung ist es an das Versorgungsgerät des elektrochirurgischen Systems angeschlossen. Bei der Anwendung des elektrochirurgischen Instruments wird ein Betriebsstromkreis geschlossen bzw. gebildet, der von einem elektrischen Anschluss des Versorgungsgeräts über eine Instrumentenelektrode des elektrochirurgischen Instruments, das behandelte Gewebe und eine weitere Elektrode - optional eine vom elektrochirurgischen Instrument separate am Patienten angebrachte Neutralelektrode - zurück zu einem Anschluss des Versorgungsgerätes führt. Die Neutralelektrode ist dann vorhanden, wenn es sich bei dem elektrochirurgischen Instrument um ein monopolares Instrument handelt. Bei einem biopolaren Instrument mit mehreren Instrumentenelektroden kann eine separate Neutralelektrode entfallen.

Das Versorgungsgerät des elektrochirurgischen Instruments stellt eine elektrische Leistung in Form einer elektrischen Betriebsspannung und/oder eines elektrischen Betriebsstromes für das elektrochirurgische Instrument zur Verfügung. Optional können auch weitere Betriebsmedien bereitgestellt werden, beispielsweise ein Betriebsfluid.

Derartige elektrochirurgische Systeme und deren Betrieb beim Behandeln von biologischem Gewebe eines Patienten sind aus dem Stand der Technik bekannt. Unterschiedliche Gewebetypen erfordern unterschiedliche Betriebseinstellungen, um eine jeweils an dem Gewebetyp angepasste Behandlung zur Verfügung zu stellen. Eine Bedienperson, insbesondere ein Operateur, kann daher am elektrochirurgischen System (z.B. am Versorgungsgerät) einen gewünschten Betriebsmodus auswählen bzw. einstellen, der auch als Mode bezeichnet wird. Dies erfordert Fachwissen der Bedienperson, um den jeweils für die vorgesehene Behandlung des Gewebes optimalen Mode einzustellen oder aus verfügbaren Modes auszuwählen.

Um einer Bedienperson die Anwendung des elektrochirurgischen Systems zu vereinfachen ist es aus dem Stand der Technik bekannt, eine automatische Erkennung des behandelten Gewebetyps durchzuführen und gegebenenfalls automatisch von einen Mode auf einen anderen Mode umzuschalten. Ein solches Verfahren zum Betreiben eines elektrochirurgischen Systems ist, zum Beispiel in DE 10 2020 105 835 A1 beschrieben. Dort wird zur Ermittlung des Gewebetyps vorgeschlagen, eine optische Gewebetyperkennung durchzuführen, weil eine Gewebetyperkennung allein basierend auf elektrischen Eigenschaften des Gewebes nur eingeschränkt möglich sei.

EP 3 876 238 A1 offenbart ein Verfahren und ein System zur Unterstützung von chirurgischen Eingriffen. Während der Behandlung werden Messdaten ermittelt und in einem Versorgungsgerät zwischengespeichert. Die zwischengespeicherten Messdaten werden dann an eine zentrale Auswertevorrichtung übertragen. In der zentralen Auswertevorrichtung werden die empfangenen Daten gespeichert und ausgewertet. Basierend auf dieser Auswertung können verfügbare Modes des Versorgungsgeräts verändert, vollständig verworfen oder neue Modes erzeugt werden.

Die durch ein Versorgungsgerät eines elektrochirurgischen Systems bereitgestellten Modes dienen unterschiedlichen Behandlungsmethoden von biologischem Gewebe und können sich daher erheblich voneinander unterscheiden. Daher muss die automatische Gewebetyperkennung mit ausreichender Sicherheit und sehr schnell erfolgen, beispielsweise wenn sich der Gewebetyp bei einer Behandlung nur kurzzeitig ändert. Dies kann zum Beispiel der Fall sein, wenn ein behandelter Gewebetyp von kleineren lokal begrenzten Strukturen eines anderen Gewebetyps durchsetzt wird.

Es kann daher als Aufgabe der vorliegenden Erfindung angesehen werden, ein elektrochirurgisches System und ein Verfahren zu dessen Betrieb zu schaffen, das schnell und sicher eine Anpassung des Betriebs an unterschiedliche Gewebetypen ermöglicht.

Diese Aufgabe wird durch ein elektrochirurgisches System mit den Merkmalen des Patentanspruches 1 sowie ein Verfahren zum Betrieb eines elektrochirurgischen Systems mit den Merkmalen des Patentanspruches 13 gelöst.

Das erfindungsgemäße elektrochirurgische System weist ein Versorgungsgerät sowie ein an das Versorgungsgerät anschließbares oder angeschlossenes elektrochirurgisches Instrument auf. Das elektrochirurgische Instrument hat wenigstens eine Instrumentenelektrode, mittels der zu behandelndes biologisches Gewebe eines Patienten beeinflusst werden kann. Die wenigstens eine Instrumentenelektrode kann dabei mit Abstand zu dem Gewebe angeordnet werden oder in Kontakt mit dem Gewebe sein. Bei der Anwendung kann sich zwischen der Instrumentenelektrode und dem Gewebe ein elektrischer Funken bilden.

Das elektrochirurgische Instrument ist insbesondere zum Schneiden und/oder Koagulieren des zu behandelnden Gewebes eingerichtet. Es kann dazu vorgesehen und eingerichtet sein, offenchirurgisch oder endoskopisch verwendet zu werden. Die wenigstens eine Instrumentenelektrode kann für die jeweilige Behandlung eine geeignete geometrische Gestalt und Dimension aufweisen, wobei es sich beispielsweise um eine nadelförmige Instrumentenelektrode oder eine spatelförmige Instrumentenelektrode handeln kann.

Das elektrochirurgische Instrument kann ein monopolares Instrument sein. Um einen Betriebsstromkreis zu schließen, wird in diesem Fall eine separate Elektrode, die als Neutralelektrode bezeichnet werden kann, elektrisch leitend am Patienten angeordnet. Ein Betriebsstrom kann dann vom Versorgungsgerät über die wenigstens eine Instrumentenelektrode des elektrochirurgischen Systems, das behandelte Gewebe und die Neutralelektrode zurück zum Versorgungsgerät fließen. Auf diese Weise wird ein geschlossener Betriebsstromkreis gebildet. Wenn es sich bei dem elektrochirurgischen Instrument um ein bipolares Instrument handelt, kann der Betriebsstromkreis durch die am elektrochirurgischen Instrument vorhandenen mehreren Instrumentenelektroden geschlossen werden, so dass eine separate Neutralelektrode entfallen kann.

Das Versorgungsgerät ist dazu eingerichtet, dem Betriebsstromkreis bzw. dem elektrochirurgischen Instrument eine Betriebsspannung und/oder einen Betriebsstrom bereitzustellen. Dabei kann entweder die Betriebsspannung oder der Betriebsstrom durch das Versorgungsgerät (z.B. einen steuerbaren Generator des Versorgungsgeräts) vorgegeben bzw. eingeprägt werden. Die Einstellungen für die Betriebsspannung und/oder den Betriebsstrom hängen dabei von einem ausgewählten bzw. eingestellten Mode des Versorgungsgeräts ab.

Jeder Mode ist durch einen Modeparametersatz mit mehreren Modeparametern charakterisiert. Eine oder mehrere der folgenden Parameter können als Modeparameter zur Bildung eines Modeparametersatzes verwendet werden: ein Maximalwert und/oder ein Mittelwert und/oder ein Effektivwert der Betriebsspannung; ein Maximalwert und/oder ein Mittelwert und/oder ein Effektivwert des Betriebsstromes; eine Modulationsart für die Betriebsspannung und/oder für den Betriebsstrom; ein Scheitelfaktor (Crest-Faktor) der Betriebsspannung und/oder des Betriebsstromes und/oder einer damit zusammenhängenden Parameters, wie z.B. einer elektrischen Betriebsleistung; eine Frequenz der Betriebsspannung und/oder des Betriebsstromes; eine Kurvenform der Betriebsspannung und/oder des Betriebsstromes, beispielsweise eine rechteckförmige, sinusförmige oder andere Kurvenform, die kontinuierlich (auch als "continuous wave" bzw. "cw" bezeichnet) oder quasikontinuierlich mit kurzen Pulsdauern und/oder Pausendauern vorgegeben sein kann; ein Gleichspannungswert der Betriebsspannung oder ein Gleichstromwert des Betriebsstromes, wobei die Gleichspannung oder der Gleichstrom kontinuierlich angelegt werden können oder entsprechend einem vorgegebenen Zeittakt ein- und ausgeschaltet werden können.

Unter einer quasikontinuierlichen Kurvenform wird insbesondere eine Kurvenform verstanden, die ein Wechselsignal, insbesondere Hochfrequenz-Wechselsignal, für die Betriebsspannung und/oder den Betriebsstrom bereitstellt, das wiederholt jeweils während einer Pulsdauer kontinuierlich anliegt (sozusagen als Puls oder Burst), wobei zwischen aufeinanderfolgenden Pulsen bzw. Pulsdauern eine Pausendauer vorhanden ist. Während der Pausendauer ist die Betriebsspannung und/oder den Betriebsstrom gleich Null oder zumindest derart vernachlässigbar klein, dass das Gewebe nicht beeinflusst wird. Die Pulsauer und die Pausendauer sind vorzugsweise gleich lang, können aber auch unterschiedlich lang sein. Die Pulsdauer und/oder die Pausendauer kann bzw. können beispielsweise maximal 10 ms oder maximal 5 ms betragen.

Das Versorgungsgerät ist erfindungsgemäß dazu eingerichtet, wenigstens einen elektrischen Betriebsparameter zu erfassen, der sich beim Betrieb des elektrochirurgischen Systems im Betriebsstromkreis ergibt. Der wenigstens eine Betriebsparameter kann ein Betriebsparameter sein, der durch das Versorgungsgerät vorgegeben ist und/oder ein Betriebsparameter, der sich bei der Verwendung in Abhängigkeit vom behandelten Gewebe und optional weiteren äußeren Bedingungen ergibt. Insbesondere werden als Betriebsparameter zumindest die Betriebsspannung und/oder der Betriebsstrom gemessen. Optional kann abhängig von der Betriebsspannung und/oder dem Betriebsstrom zusätzlich wenigstens ein weiterer Betriebsparameter berechnet, geschätzt oder anderweitig ermittelt werden. Als Betriebsparameter kann beispielsweise wenigstens einer der folgenden Parameter verwendet werden: ein ohmscher Widerstand und/oder die Impedanz des Betriebsstromkreises; ein Funkenparameter, der einen Einfluss eines Funkens zwischen der Instrumentenelektrode und dem behandelten Gewebe auf den Betriebsstrom und/oder die Betriebsspannung beschreibt; eine Wirkleistung; eine Blindleistung; eine Scheinleistung; ein Leistungsfaktor; ein beliebiger sich aus der Ermittlung der Betriebsspannung ergebender Spannungswert, beispielsweise ein Maximalwert und/oder ein Mittelwert und/oder ein Effektivwert; ein beliebiger sich aus der Messung des Betriebsstromes ergebender Stromwert, insbesondere ein Maximalwert und/oder ein Mittelwert und/oder ein Effektivwert des Betriebsstromes.

Basierend auf wenigstens einem erfassten Betriebsparameter wird geprüft, ob sich ein Gewebetyp des mittels des elektrochirurgischen Systems behandelten Gewebes von einem Primärgewebetyp, der mittels des eingestellten Modes behandelt werden soll, auf einen davon verschiedenen Sekundärgewebetyp ändert. Beispielsweise kann sich der Gewebetyp von Bindegewebe (Primärgewebetyp) zu dem Gewebe eines Blutgefäßes (Sekundärgewebetyp) ändern. Wird eine solche Änderung festgestellt, passt das Versorgungsgerät wenigstens einen Modeparameter des Modeparametersatzes zumindest temporär an, um die Veränderung des Gewebetyps von Primärgewebetyp auf den Sekundärgewebetyp zu berücksichtigen. Der anzupassende Modeparameter wird dabei beispielsweise von einem jeweils zugeordneten Primärparameterwert oder Primärzustand auf einen sich davon unterscheidenden Sekundärparameterwert oder Sekundärzustand geändert. Beispielsweise kann hierzu ein Spannungswert der Betriebsspannung und/oder ein Stromwert des Betriebsstromes und/oder eine Modulationsart der Betriebsspannung bzw. des Betriebsstromes und/oder eine Kurvenform der Betriebsspannung bzw. des Betriebsstromes verändert werden.

Die Überwachung des wenigstens einen Betriebsparameters des Betriebsstromkreises mittels des Versorgungsgeräts ist mit sehr einfachen Mitteln sehr schnell möglich, insbesondere in Zeitspannen im Millisekundenbereich, von insbesondere maximal 100 ms oder maximal 50 ms. Dadurch lässt sich ein Anpassen des wenigstens einen Modeparameters sehr schnell durchführen, um auf sehr kleine Gewebestrukturen des Sekundärgewebetyps ausreichend schnell reagieren zu können. Beispielsweise kann mittels der Erfindung sehr schnell eine Anpassung durchgeführt werden, wenn der eingestellte Mode zum Behandeln und insbesondere zum Schneiden Bindegewebe vorgesehen ist und das Bindegewebe von kleinen Blutgefäßen lokal durchsetzt ist, deren Gewebetyp (Sekundärgewebetyp) sich vom Primärgewebetyp des Bindegewebes unterscheidet.

Diese Anpassung des wenigstens einen Modeparameters wird insbesondere ausgeführt, ohne den eingestellten Mode zu verlassen und in einen anderen auswählbaren Mode umzuschalten.

Vorzugsweise weist jeder Modeparameter innerhalb eines jeweiligen Modeparametersatzes einen definierten Wertebereich auf. Es ist insbesondere vorgesehen, dass der Modeparameter diesen Wertebereich nicht verlässt. Bei der Anpassung des wenigstens einen Modeparameters innerhalb des eingestellten Modes wird der definierte Wertebereich daher nicht verlassen.

Es ist außerdem vorteilhaft, wenn das Versorgungsgerät dazu eingerichtet ist, wenigstens einen angepassten Modeparameter wieder zurück auf seine ursprüngliche Einstellung zu ändern, wenn eine erneute Änderung des Gewebetyps vom Sekundärgewebetyp auf den Primärgewebetyp festgestellt wird.

Zusätzlich oder alternativ kann eine Abbruchbedingung definiert werden, bei deren Erfüllung das Versorgungsgerät den wenigstens einen Modeparameter wieder auf seine ursprüngliche Einstellung des jeweiligen Modeparameters ändert. Eine solche Abbruchbedingung kann beispielsweise dann erfüllt sein, wenn seit dem Ändern des einen oder der mehreren Modeparameter eine vorgegebene Maximalzeitdauer abgelaufen ist. Die Maximalzeitdauer kann beispielsweise 5 Sekunden oder 2 Sekunden oder 1 Sekunde betragen. Diese optionale Maßnahme kann eine zusätzliche Sicherheit bei der Behandlung von biologischem Gewebe bieten. Der von der Bedienperson eingestellte oder ausgewählte Mode wird dabei nur kurzzeitig verändert, wenn der Sekundärgewebetyp erkannt wurde. Diese automatische Anpassung bzw. Veränderung von wenigstens einem Modeparameter kann zeitlich begrenzt werden, um der Bedienperson die Entscheidungsgewalt über die eingestellten Modeparameter zu überlassen. Das Erfüllen der Abbruchbedingung kann der Bedienperson angezeigt werden. Wenn über längere Zeit ein anderer Gewebetyp als der Primärgewebetyp behandelt werden soll, kann unter Umständen eine veränderte Modeeinstellung vorteilhaft oder notwendig sein.

Wie bereits erläutert, ist das elektrochirurgische System bei einem Ausführungsbeispiel insbesondere dazu eingerichtet, Bindegewebe als einen Gewebetyp und ein Blutgefäß als einen anderen Gewebetyp zu erkennen und/oder zumindest voneinander zu unterscheiden. Vorzugsweise kann das Bindegewebe der Primärgewebetyp und das Gewebe des Blutgefäßes der Sekundärgewebetyp sein.

Zur Erkennung des Gewebetyps, insbesondere Bindegewebe und/oder Blutgefäß, bzw. zur Erkennung der Änderung des Gewebetyps, insbesondere von Bindegewebe zu einem Blutgefäß oder umgekehrt von einem Blutgefäß zum Bindegewebe, kann vorzugsweise eine Kombination mehrerer Betriebsparameter berücksichtigt werden:
- er Widerstand oder die Impedanz des Betriebsstromkreises;
- eine Änderung des Widerstands oder er Impedanz des Betriebsstromkreises und überschreitet einen oberen Schwellenwert oder unterschreitet einen unteren Schwellenwert;
- der Funkenparameter;
- eine Änderung des Funkenparameters innerhalb einer vorgegebenen Änderungsdauer um einen Mindestwert
- der Funkenparameter überschreitet oder unterschreitet einen vorgegebenen Schwellenwert;
- der Leistungsfaktor der elektrischen Leistung des Betriebsstromkreises ändert sich um einen Mindestbetrag innerhalb einer vorgegebenen Änderungszeitdauer.

Beispielsweise kann ein Blutgefäß erkannt werden, wenn
(a) der ohmsche Widerstand des Betriebsstromkreises kleiner ist als 500 Ohm (vorzugsweise deutlich kleiner),
(b) der Funkenparameter innerhalb einer Änderungszeitdauer von vorzugsweise maximal 5 ms einen vorgegebenen Schwellenwert überschreitet und
(c) der Leistungsfaktor innerhalb eines vorgegebenen Toleranzbereichs um den Betrag von 20% liegt, beispielsweise in einem Toleranzbereich von 15% bis 25%.

Vorzugsweise kann Bindegewebe dadurch erkannt werden, dass
(a) der Widerstand des Betriebsstromkreises über einem Schwellenwert von 1500 Ohm ist,
(b) der Funkenparameter während einer Mindestzeitdauer von beispielsweise 10 ms unterhalb einem vorgegebenen Schwellenwert liegt und
(c) sich der Leistungsfaktor innerhalb einer vorgegebenen Änderungszeitdauer von beispielsweise 50 ms deutlich zwischen 1% und 100% verändert, beispielsweise um einen Prozentwert von mindestens 50% oder 60% oder 70% zunimmt oder sinkt.

Als Modeparameter kann einer oder können mehrere der folgenden Modeparameter verwendet werden:
- Maximalwert und/oder Mittelwert und/oder Effektivwert der Betriebsspannung,
- Maximalwert und/oder Mittelwert und/oder Effektivwert des Betriebsstromes,
- Wirkleistung und/oder Scheinleistung und/oder Blindleistung, die dem Betriebsstromkreis zur Verfügung gestellt wird,
- ein Leistungsfaktor, der insbesondere das Verhältnis der Wirkleistung zur Scheinleistung angibt,
- eine Frequenz- und/oder eine Kurvenform der Betriebsspannung,
- eine Frequenz- und/oder eine Kurvenform des Betriebsstromes,
- eine Modulationsart der Betriebsspannung und/oder des Betriebsstromes.

Wenn erfindungsgemäß durch die Auswertung des wenigstens einen Betriebsparameters der Übergang vom Bindegewebe als Primärgewebetyp auf ein Blutgefäß als Sekundärgewebetyp geändert wird, kann der Modeparametersatz wie folgt angepasst werden:
- Der zulässige Maximalbetrag der Betriebsspannung wird von einem Primärspannungswert auf einen Sekundärspannungswert reduziert; und/oder
- Der zulässige Maximalbetrag des Betriebsstromes wird von einem Primärstromwert auf einen Sekundärstromwert erhöht; und/oder
- Die Modulationsart wird von einer aktuell verwendeten Kurvenform (insbesondere Hochfrequenz-Wechselsignalform) für die Betriebsspannung und/oder den Betriebsstrom auf eine quasikontinuierliche Kurvenform für die Betriebsspannung und/oder den Betriebsstrom geändert.

Das Hochfrequenz-Wechselsignal ist insbesondere derart vorgegeben, dass ein Polaritätswechsel der Betriebsspannung bzw. des Betriebsstromes stattfindet. Die Betriebsspannung und/oder der Betriebsstrom kann bzw. können einen sinusförmigen Verlauf aufweisen.

Bei einem Ausführungsbeispiel wird die Anpassung des wenigstens einen Modeparameters angezeigt, beispielsweise optisch und/oder akustisch und/oder haptisch. Hierzu kann z.B. eine Bedienschnittstelle am Versorgungsgerät verwendet werden. Zusätzlich oder alternativ kann die Anzeige auch am elektrochirurgischen Instrument erfolgen, beispielsweise durch ein haptisches Signal, wie etwa ein Vibrationssignal. Einer Bedienperson kann jede automatische Änderung des Modeparameters oder der mehreren Modeparameter eines Modeparametersatzes angezeigt werden.

Während der Behandlung des Primärgewebetyps mit dem eingestellten Mode kann wenigstens einer oder genau einer der Modeparameter dynamisch an eine Gewebeimpedanz des behandelten Gewebes (Primärgewebetyp) angepasst werden. Beispielsweise kann der wenigstens eine automatisch anpassbare Modeparameter aufweisen:
- den Scheitelfaktor (Crest-Faktor) der Betriebsspannung und/oder
- den Scheitelfaktor (Crest-Faktor) des Betriebsstromes und/oder
- den Scheitelfaktor (Crest-Faktor) eines anderen elektrischen Parameters, der von der Betriebsspannung und/oder dem Betriebsstromes abhängt, wie z.B. die elektrische Betriebsleistung.

- Der Anpassungsbereich, innerhalb dessen der jeweilige Modeparameter automatisch angepasst werden kann, kann optional durch einen oberen Grenzwert und/oder einen unteren Grenzwert begrenzt sein. Zusätzlich oder alternativ können auch wenigstens ein weiterer elektrischer Parameter begrenzt werden durch einen zugeordneten oberen Grenzwert und/oder einen unteren Grenzwert, der sich als Folge der automatischen Anpassung des wenigstens einen Modeparameters ändern kann.

Irgendein hierin beschriebenes Ausführungsbeispiel eines elektrochirurgischen Systems wird erfindungsgemäß wie folgt betrieben:

Zuerst wird ein Mode eingestellt, der durch einen Modeparametersatz aus mehreren Modeparametern definiert ist. Basierend auf diesem Mode wird dem Betriebsstromkreis eine elektrische Betriebsspannung und/oder ein elektrischer Betriebsstrom durch das Versorgungsgerät vorgegeben. Während des Betriebs und insbesondere während der Behandlung von biologischem Gewebe wird wenigstens ein elektrischer Betriebsparameter des Betriebsstromkreises erfasst, insbesondere werden die Betriebsspannung und der Betriebsstrom kontinuierlich gemessen oder anderweitig ermittelt. Basierend auf der Betriebsspannung und/oder dem Betriebsstrom können ein oder mehrere weitere Betriebsparameter ermittelt werden.

Basierend auf dem wenigstens einen Betriebsparameter wird ausgewertet, ob sich der behandelte Gewebetyp ändert von einem dem eingestellten Mode zugeordneten Primärgewebetyp zu einem davon verschiedenen Sekundärgewebetyp. Ist dies der Fall, wird wenigstens ein Modeparameter des Modeparametersatzes des eingestellten Modes wenigstens temporär an den Sekundärgewebetyp angepasst.

Vorteilhafte Ausgestaltungen der Erfindung ergeben sich aus den abhängigen Patentansprüchen, der Beschreibung und der Zeichnung. Nachfolgend werden bevorzugte Ausführungsbeispiele der Erfindung anhand der beigefügten Zeichnung im Einzelnen erläutert. In der Zeichnung zeigen:

Figur 1 eine schematische, blockschaltbildähnliche Darstellung eines Ausführungsbeispiels eines elektrochirurgischen Systems,

Figur 2 eine schematische Darstellung verfügbarer Modes zum Betreiben des elektrochirurgischen Systems aus Figur 1, wobei jeder Mode einen Modeparametersatz mit mehreren Modeparametern aufweist,

Figur 3 eine Prinzipdarstellung der Verwendung eines elektrochirurgischen Instruments des elektrochirurgischen Systems aus Figur 1 bei der Behandlung von biologischem Gewebe,

Figuren 4 und 5 jeweils stark schematisierte qualitative Darstellungen von beispielhaften zeitlichen Änderungen von Betriebsparametern des elektrochirurgischen Systems aus Figur 1 und

Figur 6 stark schematisierte, beispielhafte zeitliche Verläufe von mehreren Modeparametern bei einer erkannten Änderung eines Mittels des elektrochirurgischen Instruments behandelten Gewebetyps.

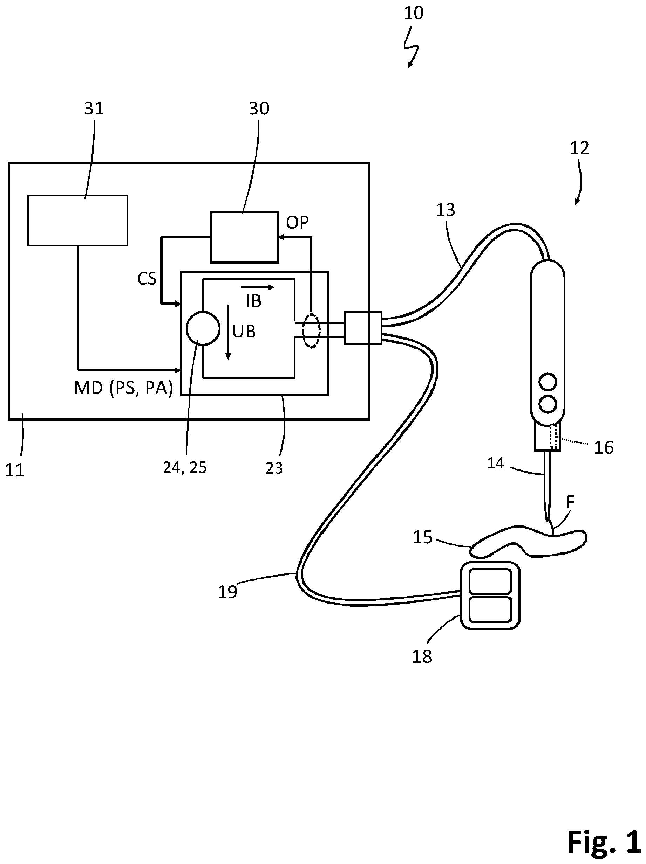

In Figur 1 ist schematisch nach Art eines Blockschaltbilds ein Ausführungsbeispiel eines elektrochirurgischen Systems 10 veranschaulicht. Das elektrochirurgische System 10 weist ein Versorgungsgerät 11 sowie eine an das Versorgungsgerät 11 angeschlossenes oder anschließbares elektrochirurgisches Instrument 12 auf. Zur elektrischen Verbindung des elektrochirurgischen Instruments 12 mit dem Versorgungsgerät 11 dient eine Leitung oder ein Kabel 13 mit wenigstens einem elektrischen Leiter. Das elektrochirurgische Instrument 12 hat wenigstens eine Instrumentenelektrode 14, mittels der auf ein zu behandelndes biologisches Gewebe 15 eines Patienten eingewirkt werden kann.

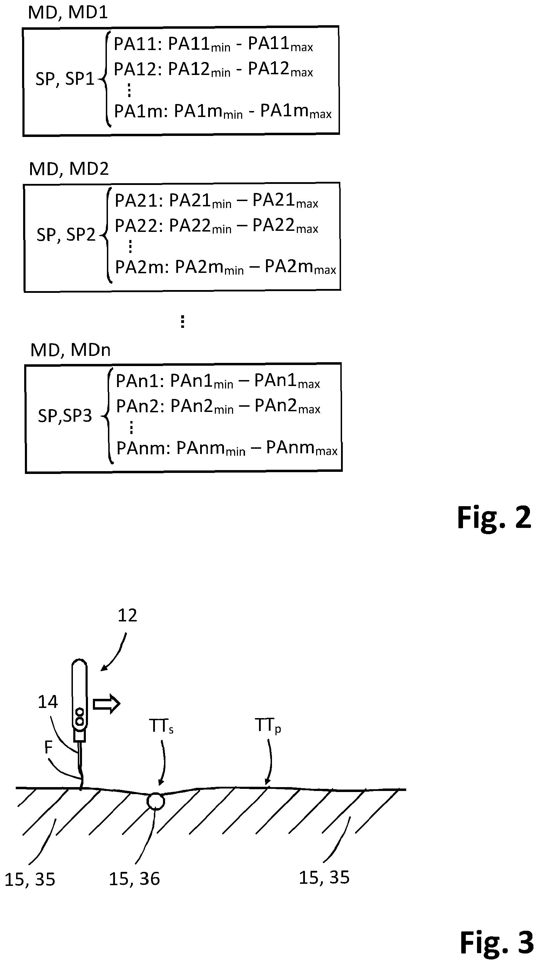

Zusätzlich zu der elektrischen Verbindung über das Kabel 13 kann zwischen dem Versorgungsgerät 11 und dem elektrochirurgischen Instrument 12 auch eine optische und/oder fluidische Verbindung hergestellt werden, wobei das Kabel 13 in diesem Fall entsprechende optische und/oder fluidische Leitungen aufweist. Beispielsweise kann an dem elektrochirurgischen Instrument 12 eine optische Erfassungseinrichtung 16 vorhanden sein, um Licht zu erfassen, das beim Einwirken auf das Gewebe 15 zwischen der Instrumentenelektrode 14 und dem Gewebe 15 entstehen kann, wenn sich zum Beispiel zwischen der Instrumentenelektrode 14 und dem Gewebe 15 ein Funke F bildet, wie es schematisch in den Figuren 1 und 3 veranschaulicht ist. Die optische Erfassungseinrichtung 16 kann mittels eines optischen Leiters des Kabels 13 mit dem Versorgungsgerät 11 verbunden sein, um dem Versorgungsgerät 11 das empfangene Licht zuzuführen.

Bei dem in Figur 1 veranschaulichten Ausführungsbeispiel ist das elektrochirurgische Instrument 12 als monopolares Instrument ausgebildet. Eine einzige Instrumentenelektrode 14 ist daher ausreichend. Zur Bildung eines geschlossenen Betriebsstromkreises 17 ist bei dem monopolaren Instrument zusätzlich zur Instrumentenelektrode 14 eine separate Elektrode vorhanden, die elektrisch leitend am Patienten angebracht ist bzw. angebracht werden kann. Diese separate Elektrode kann als Neutralelektrode 18 bezeichnet werden. Die Neutralelektrode 18 ist über eine elektrische Leitung 19 elektrisch leitend mit dem Versorgungsgerät 11 verbunden.

In Abwandlung zum veranschaulichten Ausführungsbeispiel kann es sich bei dem elektrochirurgischen Instrument 12 auch um ein bipolares Instrument handeln, das wenigstens zwei Instrumentenelektroden 14 aufweist, die unterschiedliche elektrische Potentiale aufweisen können. Der Betriebsstromkreis 17 kann dann über eine der Instrumentenelektroden 14, das Gewebe 15 und eine weitere Instrumentenelektrode des bipolaren Instruments 12 geschlossen werden. Eine separate Neutralelektrode 18 kann in diesem Fall entfallen.

Das elektrochirurgische Instrument 12 ist in der Zeichnung als Chirurgieinstrument für den offenchirurgischen Einsatz dargestellt. Alternativ hierzu kann es auch für den endoskopischen Einsatz eingerichtet sein und in diesem Fall eine Konfiguration aufweisen, so dass das elektrochirurgische Instrument 12 durch den Endoskopkanal eines Endoskops in den Patienten eingeführt werden kann.

Abhängig von der Anwendung kann die wenigstens eine Instrumentenelektrode 14 unterschiedliche Konfigurationen aufweisen. Sie kann zum Beispiel nadelförmig oder spatelförmig sein. Die wenigstens eine Instrumentenelektrode 14 kann an einem Handgriff des elektrochirurgischen Instruments 12 unbeweglich fixiert sein oder alternativ zangen- bzw. scherenartig gelagert sein, insbesondere wenn es sich um ein bipolares Instrument mit mehreren Instrumentenelektroden 14 handelt.

Das Versorgungsgerät 11 ist dazu eingerichtet, dem elektrochirurgischen Instrument 12 elektrische Energie bzw. elektrische Leistung bereitzustellen, mittels der die wenigstens eine Instrumentenelektrode 14 das biologische Gewebe 15 behandeln kann. Das elektrochirurgische Instrument 12 kann dazu eingerichtet sein, das Gewebe 15 zum Beispiel zu schneiden und/oder zu koagulieren. Hierzu weist das Versorgungsgerät 11 beim Ausführungsbeispiel einen Generator 23 auf, der eine Spannungsquelle 24 und/oder eine Stromquelle 25 aufweisen kann. Mittels des Generators 23 kann an einem Ausgang 26 des Versorgungsgeräts 11 eine Betriebsspannung UB und/oder ein Betriebsstrom IB bereitgestellt werden. Abhängig von der Ausgestaltung des Generators 23 kann dabei entweder die Betriebsspannung UB oder der Betriebsstrom IB vorgegeben bzw. eingeprägt werden. Die jeweils andere, nicht vorgegebene oder eingeprägte Größe (Betriebsstrom IB oder Betriebsspannung UB) ergibt sich dann abhängig von den elektrischen Eigenschaften des an den Ausgang 26 angeschlossenen Betriebsstromkreises 17, insbesondere dessen elektrischem Widerstand R oder dessen Impedanz.

Bei der Verwendung des elektrochirurgischen Systems 10 ist der Betriebsstromkreis 17 geschlossen, so dass ein Betriebsstrom IB fließen kann. Im Falle eines monopolaren Instruments führt er von einem Anschluss des Versorgungsgeräts 11 (bzw. des Generators 23) über einen elektrischen Leiter und im Falle eines monopolaren Instruments des Kabels 13, die Instrumentenelektrode 14, das behandelte Gewebe 15, die Neutralelektrode 18 und die elektrische Leitung 19 zurück zu einem anderen Anschluss des Versorgungsgeräts 11 (bzw. des Generators 23). Handelt es sich um ein bipolares Instrument, wird der Betriebsstromkreis 17 anstelle der Neutralelektrode 18 durch eine weitere Instrumentenelektrode 14 und einen weiteren elektrischen Leiter des Kabels 13 geschlossen.

Jedenfalls ist das behandelte Gewebe 15 Bestandteil des Betriebsstromkreises 17 und die Beschaffenheit des Gewebes 15 beeinflusst die elektrischen Eigenschaften des Betriebsstromkreises 17, beispielsweise den ohmschen Widerstand R bzw. die Impedanz des Betriebsstromkreises 17.

Das Versorgungsgerät 11 weist beispielsgemäß eine Auswerteeinheit 30 auf. Die Auswerteeinheit 30 ist dazu eingerichtet, wenigstens einen Betriebsparameter OP des Betriebsstromkreises 17 zu messen und/oder zu berechnen und/oder zu schätzen bzw. auf andere geeignete Weise zu ermitteln. Beim Ausführungsbeispiel werden mittels der Auswerteeinheit 30 die Betriebsspannung UB und/oder der Betriebsstrom IB kontinuierlich oder in regelmäßigen Zeitabständen als Betriebsparameter OP gemessen. Dabei können sowohl die Betriebsspannung UB, als auch der Betriebsstrom IB gemessen werden. Alternativ ist es auch möglich, dass die vom Generator 23 eingeprägte Größe nicht gemessen wird, da sie durch die Vorgabe des Generators 23 im Versorgungsgerät 11 bekannt ist und die vorgegebene, eingeprägte Sollgröße als Istgröße verwendet wird.

Die Auswerteeinheit 30 kann zusätzlich oder alternativ wenigstens einen Betriebsparameter OP aus einem oder mehreren der gemessenen Betriebsparameter OP ermitteln. Als Betriebsparameter OP können einer oder mehrere der nachfolgenden Parameter verwendet werden:
- ein ohmscher Widerstand R und/oder eine Impedanz und/oder der Betrag der Impedanz des Betriebsstromkreises 17,
- ein Maximalwert und/oder ein Mittelwert und/oder ein Effektivwert und/oder ein Scheitelfaktor (auch als Crest-Faktor bezeichnet) der Betriebsspannung UB und/oder des Betriebsstromes IB,
- eine dem Betriebsstromkreis 17 bereitgestellte Wirkleistung und/oder Blindleistung und/oder Scheinleistung,
- ein Leistungsfaktor, der beispielsgemäß das Verhältnis der Wirkleistung zur Scheinleistung angibt,
- ein Funkenparameter FP, der einen Einfluss des Funkens F zwischen der Instrumentenelektrode 14 des elektrochirurgischen Instruments 12 und dem behandelten Gewebe 15 auf den Betriebsstrom IB und/oder die Betriebsspannung UB beschreibt.

Der Funkenparameter FP kann aus einer Verzerrung des gemessenen Betriebsstromes IB und/oder der gemessenen Betriebsspannung UB, abgeleitet werden. Er kann zum Beispiel einen nicht-linearen Anteil der jeweils nicht durch den Generator 23 vorgegebenen oder eingeprägten elektrischen Größe (Betriebsstrom IB oder Betriebsspannung UB) beschreiben bzw. basierend darauf ermittelt werden. Dieser nicht-lineare Anteil geht maßgeblich auf den vom Funken F erzeugten nicht-linearen Widerstand zurück. Zu diesem Zweck kann die Auswerteeinheit 30 dazu eingerichtet sein, basierend auf einem linearen Ersatzschaltbild den Zusammenhang zwischen Betriebsstrom IB und Betriebsspannung UB zu ermitteln und mit dem gemessenen Betriebsstrom IB und/oder der gemessenen Betriebsspannung UB zu vergleichen. Eine dabei auftretende Differenz kennzeichnet die Nichtlinearität des Funkens F im Betriebsstromkreis 17 und kann als Basis für die Ermittlung des Funkenparameters dienen.

Beispielsweise kann die ermittelte Differenz zu der jeweils zugehörigen gemessenen Größe in Beziehung gesetzt und dadurch der Funkenparameter FP ermittelt werden. Wenn beispielsweise die Betriebsspannung UB eingeprägt ist, kann der Betriebsstrom IB gemessen werden und die Differenz aus berechnetem Wert für den Betriebsstrom IB und dem gemessenen Wert des Betriebsstromes IB berechnet werden. Ein Effektivwert dieser Differenz und ein Effektivwert des gemessenen Betriebsstromes können zueinander ins Verhältnis gesetzt und als Funkenparameter FP genutzt werden. Alternativ kann bei einem eingeprägten Betriebsstrom IB die Betriebsspannung UB zum einen berechnet und zum anderen gemessen werden und analog zu der beschriebenen Vorgehensweise der Funkenparameter FP ermittelt werden.

Als Scheitelfaktor (oder auch Crest-Faktor) für die Betriebsspannung UB bzw. für den Betriebsstrom IB wird das Verhältnis aus einem Scheitelwert zum Effektivwert verwendet. Wenn die Wechselgröße der Betriebsspannung UB bzw. des Betriebsstromes IB sinusförmig ist, entspricht der Scheitelwert der Amplitude der Wechselgröße.

Der Generator 23 ist steuerbar oder einstellbar. Die Auswerteeinheit 30 ist dazu eingerichtet, die gemessenen und/oder ermittelten Betriebsparameter OP auszuwerten. In Abhängigkeit von dem Auswerteergebnis kann die Auswerteeinheit 30 ein Steuersignal CS erzeugen, mittels dem der Betrieb des Generators 23 verändert bzw. angepasst werden kann, insbesondere eine Eigenschaft der elektrischen Betriebsspannung UB bzw. des elektrischen Betriebsstromes IB eingestellt bzw. modifiziert werden kann, wie es nachfolgend noch näher erläutert wird.

Das Versorgungsgerät 11 ist dazu eingerichtet, in unterschiedlichen Betriebsarten für unterschiedliche Anwendungen, insbesondere unterschiedliche Behandlungen von Gewebe 15, verwendet zu werden. Diese Betriebsarten werden als Modes MD bezeichnet. Eine Bedienperson, beispielsweise ein Operateur, kann einen für die geplante Anwendung des elektrochirurgischen Systems 10 geeigneten Mode MD auswählen oder einstellen. Beim Ausführungsbeispiel stellt das Versorgungsgerät 11 mehrere auswählbare Modes MD zur Verfügung, die beispielsweise mittels einer Bedienschnittstelle 31 des Versorgungsgeräts 11 ausgewählt werden können.

Der jeweils eingestellte oder ausgewählte Mode MD ist durch einen zugeordneten Modeparametersatz SP gekennzeichnet. Jeder Modeparametersatz SP besteht beim Ausführungsbeispiel aus mehreren Modeparametern PA, die wiederum den Betrieb des Generators 23 vorgeben und dadurch insbesondere charakteristische Eigenschaften der Größe und/oder des zeitlichen Verlaufs der eingeprägten Betriebsspannung UB oder des eingeprägten Betriebsstromes IB vorgeben, die bzw. der dem Betriebsstromkreis 17 bereitgestellt wird. Ein Modeparametersatz SP kann einen oder mehrere der folgenden Modeparameter PA aufweisen:
- einen Maximalwert und/oder einen Mittelwert und/oder einen Effektivwert der Betriebsspannung UB und/oder des Betriebsstromes IB,
- eine Wirkleistung und/oder eine Scheinleistung und/oder eine Blindleistung, die dem Betriebsstromkreis 17 bereitgestellt wird,
- einen Scheitelfaktor der Betriebsspannung UB und/oder des Betriebsstromes IB,
- ein Leistungsfaktor (z.B. Wirkleistung im Verhältnis zur Scheinleistung),
- eine Frequenz der Betriebsspannung UB und/oder des Betriebsstromes IB,
- eine Kurvenform der Betriebsspannung UB und/oder des Betriebsstromes IB,
- eine Modulationsart der Betriebsspannung UB und/oder des Betriebsstromes IB.

Bei der Modulationsart kann beispielsweise ein Hochfrequenz-Wechselsignal für die Betriebsspannung UB bzw. für den Betriebsstrom IB gewählt werden, das kontinuierlich (cw) oder quasikontinuierlich oder mit Unterbrechungen (mit längeren Ausschaltdauern zwischen aufeinanderfolgenden Einschaltdauern als bei der quasikontinuierlichen Kurvenform) vorgegeben werden kann.

Die Hochfrequenz-Wechselsignalform hat beim Ausführungsbeispiel einen Polaritätswechsel und ist vorzugsweise gleichanteilsfrei. Sie kann beispielsweise einen sinusförmigen Verlauf haben.

Abhängig vom eingestellten Mode kann die Frequenz des Hochfrequenz-Wechselsignals variieren und kann im Bereich von 100 kHz bis zu 4 MHz liegen, wobei sie vorzugsweise zumindest 300 kHz betragen kann.

Die Gleichsignalform hat beispielsgemäß einen Gleichanteil mit einem Betrag größer als Null und kann insbesondere ohne Polaritätswechsel des Signals ausgebildet sein. Die Gleichsignalform ist insbesondere getaktet und kann zwischen zwei und insbesondere genau zwei Werten umgeschaltet werden, insbesondere einem Gleichsignalwert ungleich Null und einem Gleichsignalwert gleich Null.

In Figur 2 sind schematisch mehrere verfügbare Modes MD1 bis MDn mit dem jeweils zugeordneten Modeparametersatz SP1 bis SPn angegeben. Für jeden Modeparameter PA kann ein zulässiger Wertebereich definiert sein. Unter Bezugnahme auf Figur 2 sind die Wertebereiche durch jeweils einen Minimalwert und jeweils einen Maximalwert veranschaulicht. Beispielsweise hat der erste Modeparameter PA11 des ersten Modeparametersatz SP1 einen Wertebereich von PA11ₘᵢₙ bis PA11ₘₐₓ. Jeder Modeparameter PA kann in seinem zugeordneten Wertebereich eingestellt bzw. modifiziert werden ohne den betreffenden Mode MD zu verlassen.

Das behandelte biologische Gewebe 15 kann abhängig von der Anwendung bzw. Art der Behandlung im Hinblick auf den behandelten Gewebetyp TT variieren. Gewebetypen TT können beispielsweise Bindegewebe 35, Gewebe eines Blutgefäßes 36, Muskelgewebe, Nervengewebe oder Epithelgewebe sein. Beispielhaft ist in Figur 3 Bindegewebe 35 und ein Blutgefäß 36 stark schematisiert veranschaulicht. Bei dem gezeigten Anwendungsbeispiel für die Gewebetypen TT von zu behandelndem biologischem Gewebe 15 stellt das Bindegewebe 35 einen Primärgewebetypen TTₚ und das Blutgefäß 36 einen Sekundärgewebetypen TTₛ dar.

Abhängig von dem zu behandelnden Gewebetyp TT (hier: Primärgewebe TTₚ) kann die Bedienperson bzw. der Operateur des elektrochirurgischen Systems 10 einen geeigneten Mode MD auswählen bzw. einstellen. Es gibt allerdings Anwendungsfälle, bei denen das behandelte Gewebe 15 nicht ausschließlich dem Primärgewebetyp TTₚ entspricht, sondern von davon abweichenden Gewebestrukturen zumindest lokal durchsetzt ist. Der aktuell verwendete Mode MD kann daher für diesen abweichenden Gewebetyp (hier: Sekundärgewebetyp TTₛ) nicht optimal geeignet sein. Lediglich beispielhaft ist in Figur 3 die Anwendung beim Schneiden von Bindegewebe 35 dargestellt, dass von im Querschnitt sehr kleinen, feinen Blutgefäßen 36 durchsetzt ist. Wenn mittels des elektrochirurgischen Instruments 12 das Bindegewebe 35 behandelt wird, beispielsweise geschnitten, werden dabei auch ein oder mehrere kleinere Blutgefäße 36 durchtrennt. Der für das Schneiden des Bindegewebes 35 ausgewählte Mode MD eignet sich aber nicht optimal für das Durchtrennen der Blutgefäße 36. Es ist daher vorgesehen, einen oder mehrere der Modeparameter PA des ausgewählten Modes MD zumindest temporär anzupassen, wenn eine Behandlung eines vom Primärgewebetyp TTₚ (z.B. Bindegewebe 35) abweichenden Sekundärgewebetyps TTₛ (z.B. Blutgefäß 36) erkannt wird. Diese Anpassung erfolgt automatisch ohne die Notwendigkeit, den Mode manuell zu verlassen oder anzupassen.

Die Anpassung des wenigstens einen Modeparameters PA ist beispielsweise derart, dass der jeweilige Modeparameter zwischen einem Primärparameterwert(oder Primärzustand) und einem Sekundärparameterwert (oder Sekundärzustand) umgeschaltet werden kann, wenn sich der Gewebetyp TT von dem Primärgewebetyp TTₚ auf den Sekundärgewebetyp TTₛ ändert bzw. umgekehrt. Abhängig davon, ob mittels des elektrochirurgischen Instruments 12 der Primärgewebetyp TTₚ oder der Sekundärgewebetyp TTₛ behandelt wird, wird der jeweils betreffende Modeparameter PA entweder auf den Primärparameterwert (oder Primärzustand) zur Behandlung des Primärgewebetyps TTₚ oder auf den Sekundärparameterwert (oder Sekundärzustand) zur Behandlung des Sekundärgewebetyps TTₛ eingestellt.

Es ist optional zu dem hier veranschaulichten Ausführungsbeispiel ebenfalls möglich, mehr als zwei unterschiedliche Gewebetypen zu unterscheiden und innerhalb eines eingestellten Modes MD zwischen drei oder mehr Gewebetypen umzuschalten und die Zusammensetzung des Modeparametersatzes PS dementsprechend zwischen drei oder mehr Kombinationen von Modeparametern PA umzuschalten. Jedem Gewebetyp TT ist dabei eine Kombination von Modeparameterwerten zugeordnet. Welche Modeparameter zur Behandlung unterschiedlicher Gewebetypen TT verändert bzw. umgeschaltet werden und die Anzahl der veränderten Modeparameter hängt von den jeweils zu behandelnden Gewebetypen TT ab, also beim Ausführungsbeispiel vom Primärgewebetyp TTₚ und vom Sekundärgewebetyp TTₛ. Es kann daher ausreichend sein, lediglich einen Modeparameter PA des betreffenden Modeparametersatzes PS zu verändern - es kann aber auch notwendig sein, mehrere der Modeparameter PA oder alle Modeparameter PA zu verändern und aneinander anzupassen.

Für diese Anpassung ist es erforderlich, die unterschiedlichen Gewebetypen TT voneinander zu unterscheiden. Deswegen ist die Auswerteeinheit 30 dazu eingerichtet, basierend auf einem Betriebsparameter OP oder einer Kombination mehrerer Betriebsparameter OP den Gewebetyp TT zu erkennen, der vom elektrochirurgischen Instrument 12 aktuell behandelt wird.

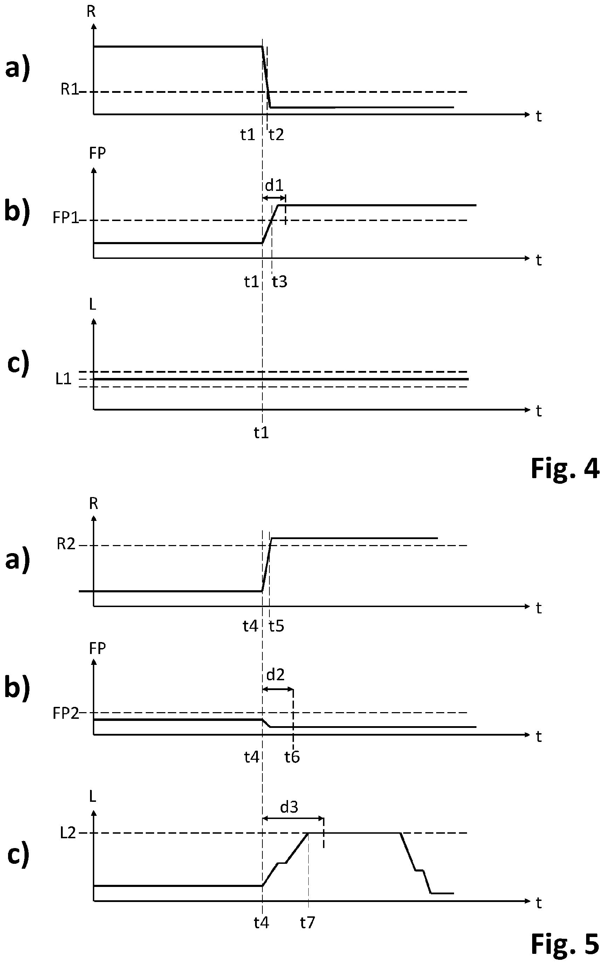

Anhand der Figuren 4-6 wird eine Gewebetyperkennung beispielhaft für den in Figur 3 dargestellten Anwendungsfall erläutert, bei dem es sich bei dem Primärgewebetyp TTₚ um Bindegewebe 35 und beim Sekundärgewebetyp TTₛ um ein Blutgefäß 36 handelt.

Wie es in den Figuren 4 und 5 veranschaulicht ist, wird zur Erkennung des Gewebetyps TT eine Kombination mehrerer Betriebsparameter OP verwendet, nämlich dem ohmschen Widerstand R des Betriebsstromkreises 17 (Figuren 4a und 5a), dem Funkenparameter FP (Figuren 4b und 5b) sowie dem Leistungsfaktor L (Figuren 4c und 5c).

Beim Ausführungsbeispiel wird das Behandeln eines Blutgefäßes 36 erkannt, wenn folgende Situation auftritt:
(a) Der ohmsche Widerstand R ist unterhalb eines ersten Widerstandsschwellenwerts R1. Der erste Widerstandsschwellenwert R1 kann beispielsweise 500 Ohm betragen.
(b) Der Funkenparameter FP ist größer als ein vorgegebener ersten Funkenparameterschwellenwert FP1. Optional kann zusätzlich oder alternativ der Gradient - beispielsweise der mittlere Gradient - der Änderung des Funkenparameters FP berücksichtigt werden, der z.B. einen Mindestbetrag aufweisen muss. Hierzu kann beispielsweise Änderungszeitdauer d1 definiert sein, innerhalb der der Funkenparameter FP ausgehend von einem Ausgangswert um einen Mindestwert angestiegen sein muss und/oder den vorgegebenen ersten Funkenparameterschwellenwert FP1 überschritten haben muss.
(c) Der Leistungsfaktor L ist zumindest nach dem Überschreiten des ersten Funkenparameterschwellenwerts FP1 und nach dem Unterschreiten des ersten Widerstandsschwellenwerts R1 im Wesentlichen konstant und bleibt innerhalb eines vorgegebenen Toleranzbandes. Dabei kann der Leistungsfaktor L beispielsweise einen ersten Leistungsfaktorwert L1 von ungefähr 0,2 (bzw. 20%) haben und hiervon lediglich innerhalb des definierten Toleranzbereichs variieren.

In den Figuren 4a-4c ist die Situation der Erkennung des Blutgefäßes 36 stark schematisiert beispielhaft veranschaulicht. Zu einem ersten Zeitpunkt t1 beginnt der ohmsche Widerstand R zu sinken und unterschreitet zu einem zweiten Zeitpunkt t2 den ersten Widerstandsschwellenwert R1. Ab oder nach dem ersten Zeitpunkt t1 beginnt der Funkenparameter FP zu steigen und überschreitet zu einem dritten Zeitpunkt t3 den ersten Funkenparameterschwellenwert FP1. Der Leistungsfaktor L bleibt beim Ausführungsbeispiel innerhalb eines in Figur 4c gestrichelt dargestellten Toleranzbereichs und ist vereinfacht als Konstante mit dem ersten Leistungsfaktorwert L1 dargestellt. Bei diesem Beispiel kann zum dritten Zeitpunkt t3 oder zumindest auf den dritten Zeitpunkt t3 folgend durch die Auswerteeinheit 30 erkannt werden, dass sich der Gewebetyp TT geändert hat und ein Blutgefäß 36 durch das elektrochirurgische Instrument 12 behandelt und beispielsgemäß geschnitten wird.

Das Behandeln und beispielsweise Schneiden von Blutgefäß 36 wird erkannt, wenn folgende Situation eintritt:
(a) Der ohmsche Widerstand R ist größer als ein zweiter Widerstandsschwellenwert R2, der beispielsweise 1500 Ohm betragen kann.
(b) Der Funkenparameter FP bleibt - zumindest während einer Mindestzeitdauer d2 von beispielsweise mindestens 50 ms - unterhalb eines zweiten Funkenparameterschwellenwerts FP2, der kleiner sein kann als der erste Funkenparameterschwellenwert FP1.
(c) Der Leistungsfaktor L ändert sich stark und weist somit innerhalb einer vorgegebenen Änderungszeitdauer d3 eine Betragsänderung auf, die einen Mindestbetrag überschreitet. Der Leistungsfaktor L kann sich bei der Behandlung von Bindegewebe in seinem gesamten Wertespektrum bis 100% ändern und hierbei große Sprünge aufweisen.

Die Erkennung von Bindegewebe 35 ist beispielhaft in Figur 5 dargestellt. Zu einem vierten Zeitpunkt t4 steigt der ohmsche Widerstand R an und überschreitet zu einem fünften Zeitpunkt t5 den zweiten Widerstandsschwellenwert R2. Der Funkenparameter FP ist bei oder nach der bekannten Widerstandsänderung während der Mindestzeitdauer d2 zwischen dem vierten Zeitpunkt t4 und einem sechsten Zeitpunkt t6) stets unterhalb des zweiten Funkenparameterschwellenwerts FP2. Nach dem vierten Zeitpunkt t4 hat der Leistungsfaktor L starke Schwankungen und Sprünge und ändert sich beispielhaft zwischen dem vierten Zeitpunkt t4 und einem siebten Zeitpunkt t7 innerhalb der Änderungszeitdauer d3 deutlich (starker Anstieg). Zum siebten Zeitpunkt t7 oder nach dem siebten Zeitpunkt t7 kann daher erkannt werden, dass mit dem elektrochirurgischen Instrument 12 Bindegewebe 35 behandelt wird.

Basierend auf der vorstehend erläuterten Erkennung des Gewebetyps TT kann entsprechend der Übergang zwischen Bindegewebe 35 und Blutgefäß 36 erkannt werden, das mittels dem elektrochirurgischen Instrument 12 behandelt wird. Es sei als Beispiel angenommen, dass mit dem eingestellten Mode MD Bindegewebe 35 behandelt werden soll. Es sei weiterhin angenommen, dass zu einem Übergangszeitpunkt tx der Übergang von Bindegewebe 35 (Primärgewebetyp TTₚ) auf das Gewebe des Blutgefäßes 36 (Sekundärgewebetyp TTₛ) erkannt wird und daraufhin eine Veränderung von einem und beispielsgemäß mehreren Modeparametern PA automatisch durch die Auswerteeinheit 30 veranlasst wird. Diese Anpassung erfolgt durch das Ansteuern des Generators 23 mittels des Steuersignals CS.

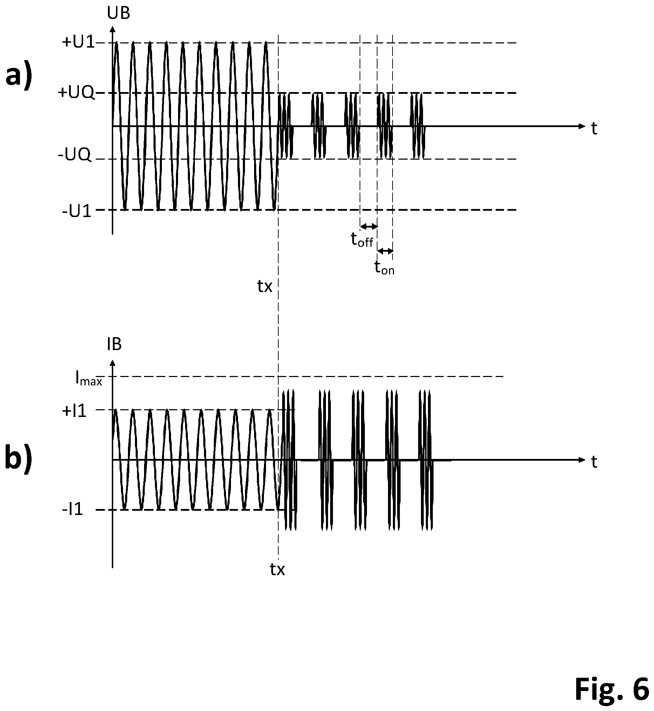

Die automatische Anpassung wenigstens eines Modeparameters PA ist beispielhaft schematisch in Figur 6 dargestellt. Vor dem Übergangszeitpunkt tx wird beim Ausführungsbeispiel Bindegewebe 35 behandelt. Zum Übergangszeitpunkt tx wird der Übergang auf ein Blutgefäß 36 erkannt.

Es sei angenommen, dass durch den Generator 23 die Betriebsspannung UB vorgegeben wird. Für die Behandlung des Bindegewebes 35 wird die Betriebsspannung UB als Hochfrequenz-Wechselsignal mit einer Amplitude U1 vorgegeben. Die Betriebsspannung UB ist sinusförmig und beispielsgemäß gleichanteilsfrei. Sie hat eine Frequenz im Bereich von mindestens 100 kHz oder mindestens 300 kHz bis zu 4 MHz (Figur 6a).

In Figur 6b ist zu erkennen, dass sich abhängig von der sinusförmigen Betriebsspannung UB bei der Behandlung des Bindegewebes (Primärgewebetyp TTₚ) ein sinusförmiger Betriebsstrom IB mit einer Amplitude I1 ergibt, der gegenüber der Betriebsspannung UB eine Phasenverschiebung aufweist. Die Phasenverschiebung kann bei der Behandlung von Bindegewebe 35 stark variieren und ist in Figur 6 lediglich der Einfachheit halber konstant dargestellt.

Wenn zum Übergangszeitpunkt tx der Übergang von Bindegewebe 35 (Primärgewebetyp TTₚ) zu dem Blutgefäß 36 (Sekundärgewebetyp TTₛ) erkannt wird, werden beim Ausführungsbeispiel folgende Modeparameter PA des Modeparametersatzes PS geändert: Die Modulationsart bzw. Kurvenform der vorgegebenen Betriebsspannung UB, der zulässige Maximalwert der Betriebsspannung UB, die Frequenz bzw. Periodendauer der Betriebsspannung UB sowie der Maximalwert des Betriebsstromes IB.

Zum Übergangszeitpunkt tx wird die Modulationsart von einer Hochfrequenz-Wechselsignalform auf eine quasikontinuierliche Kurvenform umgeschaltet. Beim Ausführungsbeispiel wird während der quasikontinuierliche Kurvenform die Hochfrequenz-Betriebsspannung UB während einer Pulsdauer tₒₙ eingeschaltet und während einer Pausendauer t_{off} ausgeschaltet. Die Amplitude UQ der HF-Wechselspannung ist kleiner als die vor dem Übergangszeitpunkt tx vorgegeben Amplitude U1 der HF-Wechselspannung. Die Pulsdauer tₒₙ bzw. Einschaltzeitdauer und die Pausendauer t_{off} bzw. Ausschaltzeitdauer sind bei diesem Ausführungsbeispiel gleich lang, insbesondere im einstelligen Millisekundenbereich, beispielsweise 5 ms. Eine Frequenz mit der das Hochfrequenz-Wechselsignal während einer quasikontinuierlichen Kurvenform ein- und ausgeschaltet wird, beträgt beispielsgemäß daher 100 Hz und ist unabhängig vom konkreten Ausführungsbeispiel vorzugsweise nicht kleiner als 50 Hz.

Der Betriebsstrom IB ist angepasst an die getaktete Betriebsspannung UB ebenfalls getaktet und kann zur Betriebsspannung UB eine konstante Phasenverschiebung aufweisen.

Die Darstellungen in den Figuren 4-6 sind stark schematisiert und lediglich qualitativ beispielhaft zu verstehen. Die zeitabhängigen Verläufe der Signale können abhängig vom Anwendungsfall variieren.

Die Erkennung des Gewebetyps TT in der Auswerteeinheit 30 basierend auf den elektrischen Betriebsparametern OP des Betriebsstromkreises 17 durch Auswertung der Beträge und/oder zeitlichen Änderung von einem oder mehreren Betriebsparametern OP kann sehr schnell durchgeführt werden, sozusagen in Echtzeit. Daher lässt sich eine Änderung des Gewebetyps TT sehr einfach und ausreichend schnell erkennen, um eine Anpassung von wenigstens einem Modeparameter PA des Modeparametersatzes PS des eingestellten Modes MD vornehmen. Beispielsweise müssen zur Unterscheidung von unterschiedlichen Gewebetypen TT lediglich maximal drei Betriebsparameter OP ermittelt und ausgewertet werden, insbesondere der ohmsche Widerstand R des Betriebsstromkreises 17, der Funkenparameter FP des Funkens F sowie der Leistungsfaktor L des Betriebsstromkreises 17. Die Berücksichtigung zusätzlicher anderer Betriebsparameter OP ist möglich, jedoch nicht erforderlich.

Die Erfindung betrifft ein elektrochirurgisches System 10 sowie ein Verfahren zu dessen Betrieb. Das elektrochirurgische System 10 weist ein Versorgungsgerät 11 sowie ein daran angeschlossenes elektrochirurgisches Instrument 12 mit wenigstens einer Instrumentenelektrode 14 auf. Mittels der Instrumentenelektrode 14 kann biologisches Gewebe 15 eines Patienten behandelt werden, beispielsweise durch Ausbildung eines zwischen der Instrumentenelektrode 14 und dem Gewebe 15 gebildeten Funkens F. Das Versorgungsgerät 11 wertet wenigstens einen Betriebsparameter OP eines Betriebsstromkreises 17 aus, zudem das elektrochirurgische Instrument 12 und das behandelte Gewebe 15 gehören und der durch das Versorgungsgerät 11 mit einer elektrischen Leistung versorgt wird. Ein Gewebetyp TT des behandelten Gewebes 15 wird durch Auswertung wenigstens eines Betriebsparameters OP erkannt. Daraufhin kann wenigstens ein elektrischer Parameter der bereitgestellten elektrischen Leistung angepasst bzw. verändert werden, so dass die dem elektrochirurgischen Instrument 12 bereitgestellte elektrische Leistung stets optimiert ist für den Gewebetyp TT des aktuell behandelten Gewebes 15.

### Bezugszeichenliste:

- 10: elektrochirurgisches System
- 11: Versorgungsgerät
- 12: elektrochirurgisches Instrument
- 13: Kabel
- 14: Instrumentenelektrode
- 15: biologisches Gewebe
- 16: optische Erfassungseinrichtung
- 17: Betriebsstromkreis
- 18: Neutralelektrode
- 19: elektrische Leitung

- 23: Generator
- 24: Spannungsquelle
- 25: Stromquelle
- 26: Ausgang des Versorgungsgeräts

- 30: Auswerteeinheit
- 31: Bedienschnittstelle

- 35: Bindegewebe
- 36: Blutgefäß

- CS: Steuersignal
- d1: Änderungszeitdauer
- d2: Mindestzeitdauer
- d3: Änderungszeitdauer
- F: Funke
- FP: Funkenparameter
- FP1: erster Funkenparameterschwellenwert
- FP2: zweiter Funkenparameterschwellenwert
- I1: Amplitude des Betriebsstroms
- IB: Betriebsstrom
- Iₘₐₓ: Maximalwert des Betriebsstroms
- L: Leistungsfaktor
- L1: erster Leistungsfaktorwert
- L2: zweiter Leistungsfaktorwert
- MD: Mode
- OP: Betriebsparameter
- PA: Modeparameter
- R: ohmscher Widerstand des Betriebsstromkreises
- R1: erster Widerstandsschwellenwert
- R2: zweiter Widerstandsschwellenwert
- SP: Modeparametersatz
- t: Zeit
- t1: erster Zeitpunkt
- t2: zweiter Zeitpunkt
- t3: dritter Zeitpunkt
- t4: vierter Zeitpunkt
- t5: fünfter Zeitpunkt
- t6: sechster Zeitpunkt
- t7: siebter Zeitpunkt
- t_{off}: Pausendauer
- tₒₙ: Pulsdauer
- tx: Übergangszeitpunkt
- TT: Gewebetyp
- TTₚ: Primärgewebetyp
- TTₛ: Sekundärgewebetyp
- U1: Amplitude der Betriebsspannung
- UB: Betriebsspannung
- UQ: Amplitude der Betriebsspannung bei quasistationärer Kurvenform

## Patentansprüche

1. Elektrochirurgisches System (10) aufweisend ein elektrochirurgisches Instrument (12) mit wenigstens einer Instrumentenelektrode (14) zur Behandlung von biologischem Gewebe (15) und ein Versorgungsgerät (11), an das das elektrochirurgisches Instrument (12) angeschlossen ist, so dass zumindest über das elektrochirurgische Instrument (12) und das behandelte Gewebe (15) ein geschlossener Betriebsstromkreis (17) gebildet werden kann,
wobei das Versorgungsgerät (11) dazu eingerichtet ist, dem elektrochirurgischen Instrument (12) eine elektrische Betriebsspannung (UB) und/oder einen elektrischen Betriebsstrom (IB) bereitzustellen, die bzw. der durch wenigstens einen einstellbaren Mode (MD) gekennzeichnet ist, und wobei jeder Mode (MD) durch einen Modeparametersatz (SP) aus mehreren Modeparametern (PA) definiert ist,
wobei das Versorgungsgerät (11) außerdem dazu eingerichtet ist, wenigstens einen elektrischen Betriebsparameter (OP) des Betriebsstromkreises (17) zu erfassen,
und wobei das Versorgungsgerät (11) außerdem dazu eingerichtet ist, basierend auf dem wenigstens einen elektrischen Betriebsparameter (OP) zu prüfen, ob sich ein mittels dem elektrochirurgischen Instrument (12) behandelter Gewebetyp (TT) von einem dem eingestellten Mode (MD) zugeordneten Primgewebetyp (TT_{P}) auf einen davon verschiedenen Sekundärgewebetyp (TTₛ) ändert, und bei einer festgestellten Änderung wenigstens einen der Modeparameter (PA) des Modeparametersatzes (SP) innerhalb des eingestellten Modes (MD) zur Anpassung an den Sekundärgewebetyp (TTₛ) zu ändern.

2. Elektrochirurgisches System nach Anspruch 1, wobei jeder Modeparameter (PA) innerhalb des jeweiligen Modeparametersatzes (SP) einen definierten Wertebereich aufweist, innerhalb dem der Wert des Modeparameters (PA) angepasst wird.

3. Elektrochirurgisches System nach Anspruch 1 oder 2, wobei das Versorgungsgerät (11) dazu eingerichtet ist, den Modeparameter (PA) wieder auf den vor der automatischen Anpassung an den Sekundärgewebetyp (TTₛ) ursprünglich eingestellten Wert zu ändern, wenn anhand des wenigstens einen elektrischen Betriebsparameters (OP) festgestellt wird, dass sich der Gewebetyp (TT) vom Sekundärgewebetyp (TTₛ) wieder auf den Primgewebetyp (Ttₚ) geändert hat.

4. Elektrochirurgisches System nach einem der vorhergehenden Ansprüche, wobei das Versorgungsgerät (11) dazu eingerichtet ist, wieder auf den vor der automatischen Anpassung an den Sekundärgewebetyp (TTₛ) ursprünglich eingestellten Wert zu ändern, wenn eine Abbruchbedingung erfüllt ist.

5. Elektrochirurgisches System nach Anspruch 4, wobei die Abbruchbedingung erfüllt ist, wenn seit dem Ändern des einen oder der mehreren Modeparameter (PA) vom jeweiligen Primärparameterwert (Vₚ) auf den jeweiligen Sekundärparameterwert (Vₛ) eine vorgegebene Maximalzeitdauer abgelaufen ist.

6. Elektrochirurgisches Systemnach einem der vorhergehenden Ansprüche, wobei das Versorgungsgerät (11) dazu eingerichtet ist, ein Bindegewebe (35) als einen Primgewebetyp (TTₚ) und ein Blutgefäß (36) als einen Sekundärgewebetyp (TTₛ) voneinander zu unterscheiden.

7. Elektrochirurgisches System nach einem der vorhergehenden Ansprüche, wobei das Versorgungsgerät (11) dazu eingerichtet ist, bei einer Erkennung der Änderung des behandelten Gewebetyps (TT) einen oder mehrere der folgenden Modeparameter (PA) zu ändern:
- Maximalwert und/oder Mittelwert und/oder Effektivwert der Betriebsspannung (UB),
- Maximalwert und/oder Mittelwert und/oder Effektivwert des Betriebsstroms (IB),
- Scheitelfaktor der Betriebsspannung (UB) und/oder des Betriebsstroms (IB),
- Wirkleitung und/oder Scheinleistung und des Betriebsstroms (IB)/oder eine Blindleistung,
- Leitungsfaktor,
- Frequenz und/oder Kurvenform der Betriebsspannung (UB),
- Frequenz und/oder Kurvenform des Betriebsstroms (IB),
- Modulationsart der Betriebsspannung (UB) und/oder des Betriebsstroms (IB).

8. Elektrochirurgisches System nach Anspruch 7 und nach Anspruch 5 oder 6, wobei das Versorgungsgerät (11) dazu eingerichtet ist, die Modeparameter (PA) wie folgt zu ändern, wenn sich der mittels des elektrochirurgischen Instruments (11) behandelte Gewebetyp (TT) von Bindegewebe (35) als Primgewebetyp (TTₚ) zu einem Blutgefäß (36) als Sekundärgewebetyp (TTₛ) des ändert:
- der zulässige Maximalbetrag der Betriebsspannung (UB) wird reduziert,
- der zulässige Maximalbetrag des Betriebsstroms (IB) wird erhöht,
- die Modulationsart wird von einer Hochfrequenz-Wechselsignalform für die Betriebsspannung (UB) und den Betriebsstrom (IB) auf eine quasikontinuierliche Kurvenform umgeschaltet.

9. Elektrochirurgisches System nach einem der vorhergehenden Ansprüche, wobei das Versorgungsgerät (11) dazu eingerichtet ist, zur Erkennung einer Änderung des behandelten Gewebetyps (T) die Betriebsspannung (UB) und den Betriebsstrom (IB) zu erfassen und einen oder mehrere der nachfolgend genannten Betriebsparameter (OP) auszuwerten:
- ohmscher Widerstand und/oder Impedanz des Betriebsstromkreises (17),
- ein Funkenparameter, der einen Einfluss eines Funkens zwischen der Instrumentenelektrode (14) des elektrochirurgischen Instruments (12) und dem behandelten Gewebe (15) auf den Betriebsstrom (IB) und/oder die Betriebsspannung (UB) beschreibt,
- Maximalwert und/oder Mittelwert und/oder Effektivwert und/oder ein Scheitelfaktor der Betriebsspannung (UB),
- Maximalwert und/oder Mittelwert und/oder Effektivwert und/oder ein Scheitelfaktor des Betriebsstroms (IB),
- Wirkleitung und/oder Blindleistung und/oder Scheinleistung und/oder Leistungsfaktor.

10. Elektrochirurgisches System nach Anspruch 9, wobei das Versorgungsgerät (11) dazu eingerichtet ist, eine Änderung vom Primgewebetyp (TTₚ) des Bindegewebes (35) zum Sekundärgewebetyp (TTₛ) des Blutgefäßes (36) dann zu erkennen, wenn sich
(i) der ohmsche Widerstand (R) reduziert,
(ii) der Funkenparameter (FP) innerhalb einer vorgegebenen Änderungszeitdauer (d1) um einen um Mindestwert erhöht oder einen vorgegebenen Funkenparameterschwellenwert überschreitet, und
(iii) der Leistungsfaktor (L) innerhalb eines vorgegebenen Toleranzbereichs bleibt.

11. Elektrochirurgisches System nach Anspruch 9 oder 10, wobei das Versorgungsgerät (11) dazu eingerichtet ist, eine Änderung vom Sekundärgewebetyp (TS) des Blutgefäßes (36) zum Primgewebetyp (TTₚ) des Bindegewebes (35) dann zu erkennen, wenn sich
(i) der ohmsche Widerstand (R) erhöht,
(ii) der Funkenparameter (FP) während einer Mindestzeitdauer unterhalb eines vorgegebenen Funkenparameterschwellenwert ist, und
(iii) der Leistungsfaktor (L) ändert sich innerhalb einer vorgegebenen Änderungszeitdauer (d3) um einen Mindestbetrag.

12. Elektrochirurgisches System nach einem der vorhergehenden Ansprüche, das dazu eingerichtet ist, einer Bedienperson das Ändern des Modeparameters (PA) oder mehrerer Modeparameter (PA) anzuzeigen.

13. Verfahren zum Betreiben eines elektrochirurgischen Systems (10) aufweisend ein elektrochirurgisches Instrument (12) mit wenigstens einer Instrumentenelektrode (14) zur Behandlung von biologischem Gewebe (15) und ein Versorgungsgerät (11), an das das elektrochirurgisches Instrument (12) angeschlossen ist, so dass zumindest über das elektrochirurgische Instrument (12) und das behandelte Gewebe (15) ein geschlossener Betriebsstromkreis (17) gebildet werden kann, wobei das Verfahren die folgenden Schritte umfasst:
- Einstellen eines Modes (MD), der durch einen Modeparametersatz (SP) aus mehreren Modeparametern (PA) definiert ist,
- Bereitstellen einer elektrischen Betriebsspannung (UB) und/oder eines elektrischen Betriebsstromes (IB) basierend auf dem eingestellten Mode (MD) für das elektrochirurgische Instrument (12) durch das Versorgungsgerät (11),
- Erfassen wenigstens einen elektrischen Betriebsparameter (OP) des Betriebsstromkreises (17),
- Prüfen, basierend auf dem wenigstens einen elektrischen Betriebsparameter (OP), ob sich ein mittels dem elektrochirurgischen Instrument (12) behandelter Gewebetyp (TT) von einem dem eingestellten Mode (MD) zugeordneten Primgewebetyp (TTₚ) auf einen davon verschiedenen Sekundärgewebetyp (TTₛ) ändert, und
- Ändern wenigstens eines der Modeparameter (PA) des Modeparametersatzes (SP) innerhalb des eingestellten Modes (MD) bei einer festgestellten Änderung des Gewebetyps (TT) von einem Primärparameterwert (Vₚ) auf einen davon verschiedenen.
